# EUROPEAN PATENT APPLICATION

(11) **EP 3 346 271 A1**
(43) Date of publication of application: **11.07.2018**
(21) Application number: 18150816.9
(22) Date of filing: 09.01.2018
(51) Int. Cl.: G01N 33/574, C07K 14/47

(54) **METHODS AND KITS FOR DETECTING CANCER**

(30) Priority: 10.01.2017 US 201762444489 P
(71) Applicant: Chang Gung Memorial Hospital, Linkou, Taoyuan City 33305 (TW)
(72) Inventor: YU, John, 33305 Taoyuan City (TW); YU, Alice L., 33305 Taoyuan City (TW)
(74) Representative: Cabinet Becker et Associés

(57) **Abstract**

Kits for detecting cancer, including an agent for measuring LRRN1 antibody and a label indicating that the reagent for measuring LRRN1 antibody is for detecting cancer are disclosed. Methods for detecting cancer in a subject, including the steps of measuring the level of LRRN1 antibody, wherein a higher LRRN1 antibody level in the subject compare to a control sample, indicates the presence of cancer, are also provided.

SEQUENCE LISTING

<110>  Chang Gung Memorial Hospital, Linkou

<120>  METHODS AND KITS FOR DETECTNG CANCER

<130>  B2650EP

<150>  US 62/444,489
<151>  2017-01-10

<160>  6

<170>  PatentIn version 3.5

<210>  1
<211>  632
<212>  PRT
<213>  Homo sapiens

<400>  1

Met Ala Arg Met Ser Phe Val Ile Ala Ala Cys Gln Leu Val Leu Gly
1               5                   10                  15

Leu Leu Met Thr Ser Leu Thr Glu Ser Ser Ile Gln Asn Ser Glu Cys
            20                  25                  30

Pro Gln Leu Cys Val Cys Glu Ile Arg Pro Trp Phe Thr Pro Gln Ser
        35                  40                  45

Thr Tyr Arg Glu Ala Thr Thr Val Asp Cys Asn Asp Leu Arg Leu Thr
    50                  55                  60

Arg Ile Pro Ser Asn Leu Ser Ser Asp Thr Gln Val Leu Leu Leu Gln
65                  70                  75                  80

Ser Asn Asn Ile Ala Lys Thr Val Asp Glu Leu Gln Gln Leu Phe Asn
                85                  90                  95

Leu Thr Glu Leu Asp Phe Ser Gln Asn Asn Phe Thr Asn Ile Lys Glu
            100                 105                 110

Val Gly Leu Ala Asn Leu Thr Gln Leu Thr Thr Leu His Leu Glu Glu
        115                 120                 125

Asn Gln Ile Thr Glu Met Thr Asp Tyr Cys Leu Gln Asp Leu Ser Asn
    130                 135                 140

Leu Gln Glu Leu Tyr Ile Asn His Asn Gln Ile Ser Thr Ile Ser Ala
145                 150                 155                 160

His Ala Phe Ala Gly Leu Lys Asn Leu Leu Arg Leu His Leu Asn Ser

165                 170                 175

Asn Lys Leu Lys Val Ile Asp Ser Arg Trp Phe Asp Ser Thr Pro Asn
        180                 185                 190

Leu Glu Ile Leu Met Ile Gly Glu Asn Pro Val Ile Gly Ile Leu Asp
        195                 200                 205

Met Asn Phe Lys Pro Leu Ala Asn Leu Arg Ser Leu Val Leu Ala Gly
    210                 215                 220

Met Tyr Leu Thr Asp Ile Pro Gly Asn Ala Leu Val Gly Leu Asp Ser
225                 230                 235                 240

Leu Glu Ser Leu Ser Phe Tyr Asp Asn Lys Leu Val Lys Val Pro Gln
                245                 250                 255

Leu Ala Leu Gln Lys Val Pro Ser Leu Lys Phe Leu Asp Leu Asn Lys
            260                 265                 270

Asn Pro Ile His Lys Ile Gln Glu Gly Asp Phe Lys Asn Met Leu Arg
        275                 280                 285

Leu Lys Glu Leu Gly Ile Asn Asn Met Gly Glu Leu Val Ser Val Asp
    290                 295                 300

Arg Tyr Ala Leu Asp Asn Leu Pro Glu Leu Thr Lys Leu Glu Ala Thr
305                 310                 315                 320

Asn Asn Pro Lys Leu Ser Tyr Ile His Arg Leu Ala Phe Arg Ser Val
                325                 330                 335

Pro Ala Leu Glu Ser Leu Met Leu Asn Asn Asn Ala Leu Asn Ala Ile
            340                 345                 350

Tyr Gln Lys Thr Val Glu Ser Leu Pro Asn Leu Arg Glu Ile Ser Ile
        355                 360                 365

His Ser Asn Pro Leu Arg Cys Asp Cys Val Ile His Trp Ile Asn Ser
    370                 375                 380

Asn Lys Thr Asn Ile Arg Phe Met Glu Pro Leu Ser Met Phe Cys Ala
385                 390                 395                 400

Met Pro Pro Glu Tyr Lys Gly His Gln Val Lys Glu Val Leu Ile Gln
                405                 410                 415

Asp Ser Ser Glu Gln Cys Leu Pro Met Ile Ser His Asp Ser Phe Pro
                420                 425                 430

Asn Arg Leu Asn Val Asp Ile Gly Thr Thr Val Phe Leu Asp Cys Arg
            435                 440                 445

Ala Met Ala Glu Pro Glu Pro Glu Ile Tyr Trp Val Thr Pro Ile Gly
    450                 455                 460

Asn Lys Ile Thr Val Glu Thr Leu Ser Asp Lys Tyr Lys Leu Ser Ser
465                 470                 475                 480

Glu Gly Thr Leu Glu Ile Ser Asn Ile Gln Ile Glu Asp Ser Gly Arg
                485                 490                 495

Tyr Thr Cys Val Ala Gln Asn Val Gln Gly Ala Asp Thr Arg Val Ala
                500                 505                 510

Thr Ile Lys Val Asn Gly Thr Leu Leu Asp Gly Thr Gln Val Leu Lys
            515                 520                 525

Ile Tyr Val Lys Gln Thr Glu Ser His Ser Ile Leu Val Ser Trp Lys
    530                 535                 540

Val Asn Ser Asn Val Met Thr Ser Asn Leu Lys Trp Ser Ser Ala Thr
545                 550                 555                 560

Met Lys Ile Asp Asn Pro His Ile Thr Tyr Thr Ala Arg Val Pro Val
                565                 570                 575

Asp Val His Glu Tyr Asn Leu Thr His Leu Gln Pro Ser Thr Asp Tyr
                580                 585                 590

Glu Val Cys Leu Thr Val Ser Asn Ile His Gln Gln Thr Gln Lys Ser
            595                 600                 605

Cys Val Asn Val Thr Thr Lys Asn Ala Ala Phe Ala Val Asp Ile Ser
    610                 615                 620

Asp Gln Glu Thr Ser Thr Ala Leu
625                 630

<210>  2
<211>  716
<212>  PRT
<213>  Homo sapiens

<400>  2

Met Ala Arg Met Ser Phe Val Ile Ala Ala Cys Gln Leu Val Leu Gly
1               5                   10                  15

Leu Leu Met Thr Ser Leu Thr Glu Ser Ser Ile Gln Asn Ser Glu Cys
            20                  25                  30

Pro Gln Leu Cys Val Cys Glu Ile Arg Pro Trp Phe Thr Pro Gln Ser
        35                  40                  45

Thr Tyr Arg Glu Ala Thr Thr Val Asp Cys Asn Asp Leu Arg Leu Thr
    50                  55                  60

Arg Ile Pro Ser Asn Leu Ser Ser Asp Thr Gln Val Leu Leu Leu Gln
65                  70                  75                  80

Ser Asn Asn Ile Ala Lys Thr Val Asp Glu Leu Gln Gln Leu Phe Asn
                85                  90                  95

Leu Thr Glu Leu Asp Phe Ser Gln Asn Asn Phe Thr Asn Ile Lys Glu
            100                 105                 110

Val Gly Leu Ala Asn Leu Thr Gln Leu Thr Thr Leu His Leu Glu Glu
        115                 120                 125

Asn Gln Ile Thr Glu Met Thr Asp Tyr Cys Leu Gln Asp Leu Ser Asn
    130                 135                 140

Leu Gln Glu Leu Tyr Ile Asn His Asn Gln Ile Ser Thr Ile Ser Ala
145                 150                 155                 160

His Ala Phe Ala Gly Leu Lys Asn Leu Leu Arg Leu His Leu Asn Ser
                165                 170                 175

Asn Lys Leu Lys Val Ile Asp Ser Arg Trp Phe Asp Ser Thr Pro Asn
            180                 185                 190

Leu Glu Ile Leu Met Ile Gly Glu Asn Pro Val Ile Gly Ile Leu Asp
        195                 200                 205

Met Asn Phe Lys Pro Leu Ala Asn Leu Arg Ser Leu Val Leu Ala Gly
    210                 215                 220

Met Tyr Leu Thr Asp Ile Pro Gly Asn Ala Leu Val Gly Leu Asp Ser
225                 230                 235                 240

Leu Glu Ser Leu Ser Phe Tyr Asp Asn Lys Leu Val Lys Val Pro Gln
                245                 250                 255

Leu Ala Leu Gln Lys Val Pro Ser Leu Lys Phe Leu Asp Leu Asn Lys
            260                 265                 270

Asn Pro Ile His Lys Ile Gln Glu Gly Asp Phe Lys Asn Met Leu Arg
        275                 280                 285

Leu Lys Glu Leu Gly Ile Asn Asn Met Gly Glu Leu Val Ser Val Asp
    290                 295                 300

Arg Tyr Ala Leu Asp Asn Leu Pro Glu Leu Thr Lys Leu Glu Ala Thr
305                 310                 315                 320

Asn Asn Pro Lys Leu Ser Tyr Ile His Arg Leu Ala Phe Arg Ser Val
                325                 330                 335

Pro Ala Leu Glu Ser Leu Met Leu Asn Asn Asn Ala Leu Asn Ala Ile
            340                 345                 350

Tyr Gln Lys Thr Val Glu Ser Leu Pro Asn Leu Arg Glu Ile Ser Ile
        355                 360                 365

His Ser Asn Pro Leu Arg Cys Asp Cys Val Ile His Trp Ile Asn Ser
    370                 375                 380

Asn Lys Thr Asn Ile Arg Phe Met Glu Pro Leu Ser Met Phe Cys Ala
385                 390                 395                 400

Met Pro Pro Glu Tyr Lys Gly His Gln Val Lys Glu Val Leu Ile Gln
                405                 410                 415

Asp Ser Ser Glu Gln Cys Leu Pro Met Ile Ser His Asp Ser Phe Pro
            420                 425                 430

Asn Arg Leu Asn Val Asp Ile Gly Thr Thr Val Phe Leu Asp Cys Arg
        435                 440                 445

Ala Met Ala Glu Pro Glu Pro Glu Ile Tyr Trp Val Thr Pro Ile Gly
    450                 455                 460

Asn Lys Ile Thr Val Glu Thr Leu Ser Asp Lys Tyr Lys Leu Ser Ser
465                 470                 475                 480

Glu Gly Thr Leu Glu Ile Ser Asn Ile Gln Ile Glu Asp Ser Gly Arg
                485                 490                 495

Tyr Thr Cys Val Ala Gln Asn Val Gln Gly Ala Asp Thr Arg Val Ala
            500                 505                 510

Thr Ile Lys Val Asn Gly Thr Leu Leu Asp Gly Thr Gln Val Leu Lys
        515                 520                 525

Ile Tyr Val Lys Gln Thr Glu Ser His Ser Ile Leu Val Ser Trp Lys
    530                 535                 540

Val Asn Ser Asn Val Met Thr Ser Asn Leu Lys Trp Ser Ser Ala Thr
545                 550                 555                 560

Met Lys Ile Asp Asn Pro His Ile Thr Tyr Thr Ala Arg Val Pro Val
                565                 570                 575

Asp Val His Glu Tyr Asn Leu Thr His Leu Gln Pro Ser Thr Asp Tyr
            580                 585                 590

Glu Val Cys Leu Thr Val Ser Asn Ile His Gln Gln Thr Gln Lys Ser
        595                 600                 605

Cys Val Asn Val Thr Thr Lys Asn Ala Ala Phe Ala Val Asp Ile Ser
    610                 615                 620

Asp Gln Glu Thr Ser Thr Ala Leu Ala Ala Val Met Gly Ser Met Phe
625                 630                 635                 640

Ala Val Ile Ser Leu Ala Ser Ile Ala Val Tyr Phe Ala Lys Arg Phe
                645                 650                 655

Lys Arg Lys Asn Tyr His His Ser Leu Lys Lys Tyr Met Gln Lys Thr
            660                 665                 670

Ser Ser Ile Pro Leu Asn Glu Leu Tyr Pro Pro Leu Ile Asn Leu Trp
        675                 680                 685

Glu Gly Asp Ser Glu Lys Asp Lys Asp Gly Ser Ala Asp Thr Lys Pro
    690                 695                 700

Thr Gln Val Asp Thr Ser Arg Ser Tyr Tyr Met Trp
705                 710                 715

<210>  3
<211>  37
<212>  DNA
<213>  Artificial Sequence

<220>
<223>  A forward primer nucleotide sequence for amplifying full-length
       human LRRN1

<400>  3
gatcggatcc atggctagga tgagctttgt tatagca                                37


<210>  4
<211>  39
<212>  DNA
<213>  Artificial Sequence

<220>
<223>  A reverse primer nucleotide sequence for amplifying full-length
       human LRRN1

<400>  4
gatcctcgag ttaccacatg taatagcttc tggatgtgt                              39


<210>  5
<211>  37
<212>  DNA
<213>  Artificial Sequence

<220>
<223>  A sense primer for subcloning the extracellular domain of LRRN1
       in pEF1/Myc-His plasmid

<400>  5
gatcggatcc atggctagga tgagctttgt tatagca                                37


<210>  6
<211>  36
<212>  DNA
<213>  Artificial Sequence

<220>
<223>  An antisense primer for subcloning the extracellular domain of
       LRRN1 in pEF1/Myc-His plasmid

<400>  6
gatcctcgag aagggctgta ctggtttctt gatcag                                 36

## Description

### BACKGROUND OF THE INVENTION

Reliable detection of small cancer mass, as well as early cancer, is difficult. The vast majority of patients with cancer are presently diagnosed at a late stage when the patients are symptomatic or cancer has already extended outside of the capsule to invade surrounding organs and/or has metastasized extensively. This is significant, since late detection of cancer almost always results in low survival rate.

A number of laboratories have developed diagnostic tests based upon the release of one or more tumor-associated antigens (TAAs) into the bloodstream, as well as the detection of one or more TAAs within biopsy specimens (e.g., the immunoassay for CA-125 for the diagnosis of ovarian cancer). However, these TAAs have not been successfully employed for early detection and/or diagnosis of cancer.

There is an unmet need for an economical and accurate laboratory diagnostic test for cancer and the present invention satisfy this and other needs.

### BRIEF SUMMARY OF THE INVENTION

In one embodiment, the present invention discloses methods for detecting cancer in a subject, comprising the steps of measuring the level of an antibody that binds to a polypeptide in the sample of the subject, wherein the polypeptide comprises an amino acid sequence at least 90% homologous to SEQ ID NO:1, and wherein an increase of the antibody level in comparison to the antibody level in a cancer free sample, indicates the presence of cancer in the subject.

In another embodiment, the present invention discloses methods for detecting pancreatic cancer in a subject, comprising the steps of measuring the following biomarkers in the sample of the subject: (a) the level of an antibody that binds to a polypeptide, wherein the polypeptide comprises an amino acid sequence at least 90% homologous to SEQ ID NO:1; and (b) CA 19-9, wherein an increase of the antibody level and/or an increase of CA 19-9 in comparison to the corresponding antibody and CA 19-9 levels in a pancreatic cancer free sample, indicates the presence of pancreatic cancer in the subject.

In yet another embodiment, the present invention discloses methods for detecting breast cancer in a subject, comprising the steps of measuring the following biomarkers in the sample of the subject: (a) the level of an antibody that binds to a polypeptide, wherein the polypeptide comprises an amino acid sequence at least 90% homologous to SEQ ID NO:1; and (b) CA 15-3, wherein an increase of the antibody level and/or an increase of CA 15-3 in comparison to the corresponding antibody and CA 15-3 levels in a breast cancer free sample, indicates the presence of breast cancer in the subject.

In another embodiment, method for detecting pancreatitis in a subject, comprising the steps of measuring in the sample of the subject an antibody that binds to a polypeptide, wherein the polypeptide comprises an amino acid sequence at least 90% homologous to SEQ ID NO:1, and wherein an increase level of the antibody in comparison to the corresponding antibody level in a pancreatitis free sample, is indicative the subject having pancreatitis

The present invention also discloses kits for detecting cancer in a subject, comprising an agent for detecting or measuring an antibody that binds to a polypeptide, wherein the polypeptide comprises an amino acid sequence at least 90% homologous to SEQ ID NO:1. In one embodiment, the kit further comprises a label indicates that the agent for measuring the antibody level is for detecting cancer.

The present invention further discloses kits for detecting pancreatitis in a subject, comprising an agent for detecting or measuring an antibody that binds to a polypeptide, wherein the polypeptide comprises an amino acid sequence at least 90% homologous to SEQ ID NO:1. In one embodiment, the kit further comprises a label indicates that the agent for measuring the antibody level is for detecting pancreatitis.

Also provided are agents for detecting or measuring an antibody in the manufacture of a kit for detecting cancer in a subject, wherein the antibody binds to a polypeptide comprises an amino acid sequence at least 90% homologous to SEQ ID NO:1.

The subject matter should be understood by reference to appropriate portions of the entire specification, any or all drawings and each claim.

The invention will become more apparent when read with the accompanying figures and detailed description which follow.

### BRIEF DESCRIPTION OF THE DRAWINGS

Illustrative embodiments of the present invention are described in detail below with reference to the following Figures:
FIG 1 is a Western Blot image illustrating the expression of ALPL (alkaline phosphatase), CD133 and leucine-rich repeat neuronal protein 1 (LRRN1) in human embryonic stem cells (hESCs) and Embryoid Body (EB).
FIG. 2 illustrates schematically the presence of LRRN1 on the surface of the cancer stem cells and the binding of LRRN1 antibody to LRRN1.
FIG. 3 illustrates schematically the detection of LRRN1 antibody in the serum using an indirect ELISA assay.
Fig. 4A is a bar graph illustrating the serum LRRN1 antibody level in 40 pancreatic cancer patients and 40 control patients (without pancreatic cancer).
Fig. 4B is a bar graph illustrating the serum CA19-9 level in 40 pancreatic cancer patients and 40 control patients (without pancreatic cancer).
Fig. 4C is an ROC curve illustrating the AUC of LRRN1 antibody, the AUC of CA19-9 and the AUC of LRRN1 antibody combined with CA19-9 in pancreatic cancer.
Fig. 5A and Fig. 5B are ROC curves illustrating the AUC of LRRN1 antibody in cholangiocarcinoma and gallbladder cancer, respectively.
Fig. 6A and Fig. 6B are bar graphs illustrating the serum LRRN1 antibody levels in gallbladder cancer (Fig. 6A) or bile duct cancer (Fig. 6B) and gallstone, intrahepatic duct (IHD) stone and common bile duct (CBD) stone.
Fig. 7A is a bar graph illustrating the serum LRRN1 antibody level in patients with breast cancer and in patients without breast cancer (control).
Fig. 7B is an ROC curve illustrating the AUC of LRRN1 antibody for breast cancer diagnosis.
Fig. 7C is a bar graph illustrating the sensitivity of LRRN1 antibody, CA15-3, and the combination thereof in different stages (stage 0 to stage III) of breast cancer and all breast cancer patients.

### DETAILED DESCRIPTION OF THE INVENTION

As used herein, the articles "a" and "an" refer to one or more than one (*i.e.,* at least one) of the grammatical object of the article.

The term "subject" may refer to a vertebrate suspected of having cancer or pancreatitis. Subjects include warm-blooded animals, such as mammals, such as a primate, and, more preferably, a human. Non-human primates are subjects as well. The term subject includes domesticated animals, such as cats, dogs, etc., livestock (for example, cattle, horses, pigs, sheep, goats, etc.) and laboratory animals (for example, mouse, rabbit, rat, gerbil, guinea pig, etc.).

The term "antibody" as used herein refers to immunoglobulin molecules and immunologically active portions of immunoglobulin molecules, i.e., molecules that contain an antigen binding site that immunospecifically binds a polypeptide. As such, the term antibody encompasses not only whole antibody molecules, but also antibody fragments as well as variants (including derivatives) of antibodies and antibody fragments. In natural antibodies, two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the polypeptide. The light and heavy chains of an immunoglobulin each have three CDRs, designated L-CDR1, L-CDR2, L-CDR3 and H-CDR1, H-CDR2, H-CDR3, respectively. An antigen-binding site, therefore, includes six CDRs, comprising the CDR set from each of a heavy and a light chain V region. Framework Regions (FRs) refer to amino acid sequences interposed between CDRs.

Identity or homology with respect to a specified amino acid sequence of this invention is defined herein as the percentage of amino acid residues in a candidate sequence that are identical with the specified residues, after aligning the sequences and introducing gaps, if necessary, to achieve the maximum percent homology, and not considering any conservative substitutions as part of the sequence identity. None of N-terminal, C-terminal or internal extensions, deletions, or insertions into the specified sequence shall be construed as affecting homology. Methods of alignment of sequences for comparison are well known in the art. While such alignments may be done by hand using conventional methods, various programs and alignment algorithms are described in: Smith and Waterman, Adv. Appl. Math. 2:482, 1981; Needleman and Wunsch, J. Mol. Biol. 48:443, 1970; Pearson and Lipman, Proc. Natl. Acad. Sci. U.S.A. 85:2444, 1988; Higgins and Sharp, Gene 73:237, 1988; Higgins and Sharp, CABIOS 5:151, 1989; Corpet et al, Nucleic Acids Research 16:10881, 1988; and Pearson and Lipman, Proc. Natl. Acad. Sci. U.S.A. 85:2444, 1988. Altschul et al., Nature Genet. 6:119, 1994, presents a detailed consideration of sequence alignment methods and homology calculations. The NCBI Basic Local Alignment Search Tool (BLAST (Altschul et al, J. Mol. Biol. 215:403, 1990) is available from several sources, including the National Center for Biotechnology Information (NCBI, Bethesda, Md.) and on the internet, for use in connection with the sequence analysis programs blastp, blastn, blastx, tblastn and tblastx. A description of how to determine sequence identity using this program is available on the NCBI website.

All numbers herein may be understood as modified by "about." In one embodiment, the term "about," when referring to a measurable value such as an amount, a temporal duration, and the like, is meant to encompass variations of ± 10%, preferably ± 5%, more preferably ± 1%, and even more preferably ± 0.1% from the specified value, as such variations are appropriate to the sensitivity of LRRN1 for pancreatic cancer diagnosis, unless other specified. As used herein, the term "about," when referring to a range, is meant to encompass variations of ± 10% within the difference of the range, preferably ± 5%, more preferably ± 1%, and even more preferably ± 0.1% from the specified value, as such variations are appropriate to the AUC ofLRRN for pancreatic cancer diagnosis, unless other specified.

### METHODS FOR DIAGNOSING CANCER

There has been a growing interest in using cancer-associated autoantibodies as serological cancer biomarkers. The persistence and stability of cancer-associated autoantibodies in the serum of cancer patients is an advantage over other potential markers, including the TAAs themselves, some of which are released by tumors but rapidly degrade or are cleared after circulating in the serum for a limited time. However, in contrast to autoimmune diseases, where the presence of an individual autoantibody may have diagnostic value, cancer-associated autoantibodies, when evaluated individually, have little diagnostic value, primarily because of their low frequency.

Specific glycoproteins are expressed in undifferentiated human embryonic stem cells (hESCs), which is defined as a group of cells at the early stage of embryonic development, with the capacity for self-renewal and differentiation to generate different types of cells and tissues.

In one embodiment, the glycoproteins are expressed in hESCs but their expression declines dramatically upon differentiation of hESCs to form three germ layers/mature tissue, and once again can be found on the surface of cancer stem cells. This is the so-called tumor specific antigen (TSA), to which the human body recognizes as foreign and produces antibody against it. The anti-TSA antibodies appear *de novo* with cancer and can be used as a serological cancer biomarker for cancer diagnosis. In another embodiment, the glycoproteins exist in the normal tissue that pre-date malignancy, as well as in cancer. This is the so-called tumor associated antigen (TAA), which rarely elicit cellular and/or humoral immune responses specific for the cancer. The anti-TAA autoantibodies are present before and persisting into the cancer phase of illness and are not useful biomarker for cancer.

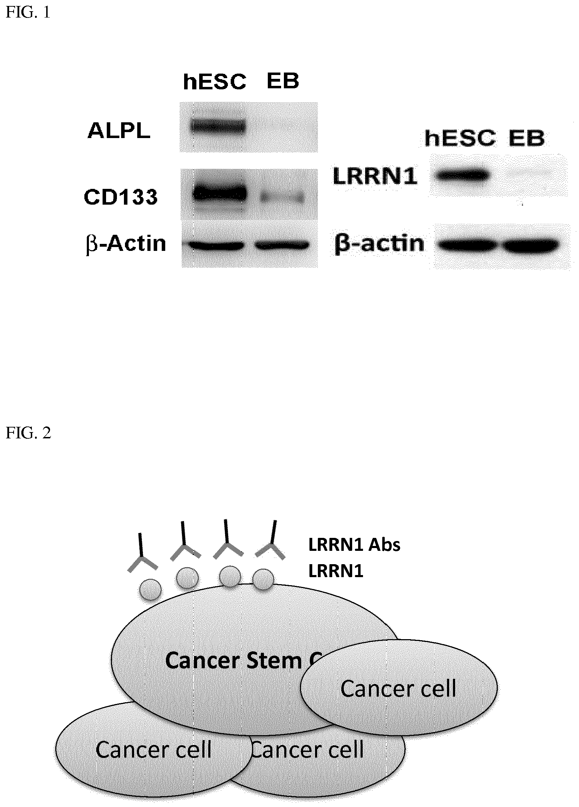

The present invention is based, in part, on the identification of particular N-linked sialylated glycoproteins as tumor specific glycoproteins. As illustrated in Fig. 1, one of the tumor specific sialylated N-glycoproteins, leucine-rich repeat neuronal protein 1 (LRRN1), is many folds higher on the surface of cancer stem cells as compared to that of EB. The higher expression of LRRN1 postnatally, on cancer stem cells, elicits an immune response and the formation of LRRN1 antibody in the plasma of patients with cancer, see Fig. 2.

In breast cancer, the expression level of LRRN1 is 6-10 folds higher in cancer stem cells (CSC) as compared to non-CSC, implying LRRN1 may play a role in carcinogenesis.

LRRN1 comprises a leucine-rich repeat domain, an immunoglobin-like domain and Fibronectin type III domain. In one embodiment, LRRN1 comprises an amino acid sequence at least 90%, 91%, 92%, 93%, 94%, 95%, 96%, 97%, 98%, 99% or 100% homologous to SEQ ID NO: 1 (extracellular domain of LRRN1).
SEQ ID NO: 1 
MARMSFVIAACQLVLGLLMTSLTESSIQNSECPQLCVCEIRPWFTPQSTYREATTVDCND
LRLTRIPSNLSSDTQVLLLQSNNIAKTVDELQQLFNLTELDFSQNNFTNIKEVGLANLTQL
TTLHLEENQITEMTDYCLQDLSNLQELYINHNQISTISAHAFAGLKNLLRLHLNSNKLKVI
DSRWFDSTPNLEILMIGENPVIGILDMNFKPLANLRSLVLAGMYLTDIPGNALVGLDSLES
LSFYDNKLVKVPQLALQKVPSLKFLDLNKNPIHKIQEGDFKNMLRLKELGINNMGELVS
VDRYALDNLPELTKLEATNNPKLSYIHRLAFRSVPALESLMLNNNALNAIYQKTVESLPN
LREISIHSNPLRCDCVIHWINSNKTNIRFMEPLSMFCAMPPEYKGHQVKEVLIQDSSEQCL
PMISHDSFPNRLNVDIGTTVFLDCRAMAEPEPEIYWVTPIGNKITVETLSDKYKLSSEGTL
EISNIQIEDSGRYTCVAQNVQGADTRVATIKVNGTLLDGTQVLKIYVKQTESHSILVSWKV
 
NSNVMTSNLKWSSATMKIDNPHITYTARVPVDVHEYNLTHLQPSTDYEVCLTVSNIHQQ
TQKSCVNVTTKNAAFAVDISDQETSTAL
 In another embodiment, LRRN1 comprises an amino acid sequence at least 90%, 91%, 92%, 93%, 94%, 95%, 96%, 97%, 98%, 99% or 100% homologous to SEQ ID NO: 2 (the full length LRRN1).
SEQ ID NO:2

MARMSFVIAACQLVLGLLMTSLTESSIQNSECPQLCVCEIRPWFTPQSTYREATTVDCND
LRLTRIPSNLSSDTQVLLLQSNNIAKTVDELQQLFNLTELDFSQNNFTNIKEVGLANLTQL
TTLHLEENQITEMTDYCLQDLSNLQELYINHNQISTISAHAFAGLKNLLRLHLNSNKLKVI
DSRWFDSTPNLEILMIGENPVIGILDMNFKPLANLRSLVLAGMYLTDIPGNALVGLDSLES
LSFYDNKLVKVPQLALQKVPSLKFLDLNKNPIHKIQEGDFKNMLRLKELGINNMGELVS
VDRYALDNLPELTKLEATNNPKLSYIHRLAFRSVPALESLMLNNNALNAIYQKTVESLPN
LREISIHSNPLRCDCVIHWINSNKTNIRFMEPLSMFCAMPPEYKGHQVKEVLIQDSSEQCL
PMISHDSFPNRLNVDIGTTVFLDCRAMAEPEPEIYWVTPIGNKITVETLSDKYKLSSEGTL
EISNIQIEDSGRYTCVAQNVQGADTRVATIKVNGTLLDGTQVLKIYVKQTESHSILVSWKV
NSNVMTSNLKWSSATMKIDNPHITYTARVPVDVHEYNLTHLQPSTDYEVCLTVSNIHQQ
TQKSCVNVTTKNAAFAVDISDQETSTALAAVMGSMFAVISLASIAVYFAKRFKRKNYHHS
LKKYMQKTSSIPLNELYPPLINLWEGDSEKDKDGSADTKPTQVDTSRSYYMW

The present invention is directed to methods for detecting cancer in a subject, comprising the steps of measuring the level of an LRRN1 antibody, and wherein an increase of the LRRN1 antibody level in comparison to the LRRN1 antibody level in a cancer free sample, indicates the presence of cancer in the subject.

In one embodiment, LRRN1 antibody is an antibody that bind to a polypeptide comprising an amino acid sequence at least 90%, 91%, 92%, 93%, 94%, 95%, 96%, 97%, 98%, 99% or 100% homologous to SEQ ID NO: 1. In another embodiment, LRRN1 antibody is an antibody that bind to a polypeptide comprising an amino acid sequence at least 90%, 91%, 92%, 93%, 94%, 95%, 96%, 97%, 98%, 99% or 100% homologous to SEQ ID NO: 2.

In an exemplary embodiment, the LRRN1 antibody is detected in the plasma of patient with pancreatic cancer, cholangiocarcinoma (bile duct cancer), gall bladder cancer or breast cancer.

Measurement of the LRRN1 antibody in a sample may easily be carried out by immunoassay using a polypeptide comprising an amino acid sequence at least 90%, 91%, 92%, 93%, 94%, 95%, 96%, 97%, 98%, 99% or 100% homologous to SEQ ID NO: 1 or SEQ ID NO: 2 as an antigen. Immunoassays per se are well-known in the art, and includes, when classified based on the reaction mode, sandwich method, competition method, agglutination method, Western blot method and the like. When classified based on the label, immunoassays include radioimmunoassay, fluorescence immunoassay, enzyme immunoassay, biotin immunoassay and the like, and the immunoassay of the LRRN1 antibody may be carried out by any of these immunoassays.

In cases where enzymes are used as a label of antibodies, the enzyme is not particularly restricted as long as it satisfies such conditions that the turnover number is large, that the enzyme is stable even when it is bound to an antibody, that it specifically colors its substrate and the like. For example, enzymes used in an ordinary enzyme immunoassay such as peroxidase, ß-galactosidase, alkaline phosphatase, glucose oxidase, acetylcholinesterase, glucose-6-phosphate dehydrogenase, and malate dehydrogenase may be used. Enzyme inhibitors, coenzymes and the like may also be used. Binding of these enzymes with an antibody may be carried out by a known method using a cross-linking agent such as a maleimide compound. As a substrate, known substances may be used depending on the kind of the used enzyme. For example, in cases where peroxidase is used as an enzyme, 3,3',5,5'-tetramethylbenzidine may be used; and in cases where alkaline phosphatase is used as an enzyme, para-nitrophenol or the like may be used. As a radioisotope, those used in an ordinary radioimmunoassay such as ¹²⁵I and ³H may be used. As a fluorescent dye, one used in an ordinary fluorescent antibody technique such as fluorescein isothiocyanate (FITC), tetramethylrhodamine isothiocyanate (TRITC) or the like may be used.

Briefly, in sandwich immunoassays, for example, the polypeptide used as an antigen (e.g., LRRN1 with 90% homology to SEQ ID No:1 or SEQ ID NO:2) is immobilized on a solid phase, and then reacted with a sample such as a serum. After washing the solid phase, the resultant is reacted with an appropriate secondary antibody. After washing the solid phase, the secondary antibody bound to the solid phase is measured. In the method for detecting a cancer(s) according to the present invention, it is preferred to immobilize an antigen polypeptide on a solid phase, because immobilization on a solid phase makes it possible to easily remove the unbound secondary antibody. The secondary antibody bound to a solid phase may be measured by labeling the secondary antibody with a labeling substance exemplified above. The thus measured amount of the secondary antibody corresponds to the amount of the LRRN1 antibody in a serum sample. In cases where an enzyme is used as a labeling substance, the amount of the antibody may be measured by adding a substrate which is decomposed by the enzymatic activity to develop a color, and then optically measuring the amount of decomposed substrate. In cases where a radioisotope is used as a labeling substance, the amount of radiation from the radioisotope may be measured with a scintillation counter or the like.

Measurement of the antibody in a sample may easily be carried out by a well-known immunoassay. Specifically, for example, LRRN1 antibody which may exist in a sample may be measured by preparing a polypeptide comprises an amino acid sequence at least 90% 91%, 92%, 93%, 94%, 95%, 96%, 97%, 98%, 99% or 100% homologous to SEQ ID No:1 or SEQ ID NO: 2 which immunologically reacts with LRRN1 antibody, and then carrying out an immunoassay using the prepared antibody or fragment thereof.

The detection method of the present invention may be carried out in combination with other cancer antigens and/or cancer markers so that the detection accuracy of cancers can be more improved. For example, the method of the present invention may be carried out in combination with diagnosis using known cancer markers. In one embodiment, the detection of serum LRRN1 antibody can be carried out in combination with the measurement of CA 19-9 in a subject suspect of having pancreatic cancer. Advantageously, this combination has additive or synergistic effects on pancreatic cancer diagnosis. In another embodiment, the detection of serum LRRN1 antibody can be carried out in combination with the measurement of CA 15-3 in a subject suspect of having breast cancer. Advantageously, this combination has additive or synergistic effects on breast cancer diagnosis. By the detection method of the present invention, cancers in a living body can be detected, including an invisible small cancer or a cancer which exists in a deep part of a body, and thus the method is useful for early detection of cancers. Further, by applying the detection method of the present invention to patients in the follow-up period after cancer therapy, cancer recurrence, if any, can be detect in its early stage.

The more cancer cells expressing LRRN1 in a subject, the more polypeptides and mRNAs encoding LRRN1 accumulate in the body, which causes an increased amount of LRRN1 antibodies in the serum. On the other hand, if the cancer load decreases, there are less polypeptides and mRNAs encoding LRRN1 in the subject, which causes a decreased amount of LRRN1 antibodies in the serum. Thus, if the expression level of LRRN1 polypeptide is high, it can be determined that cancer growth, recurrence and/or metastasis has occurred, i.e., the stage of progression of cancer is advanced. Hence, cancer progression can be detected by the method of the present invention.

### KITS FOR DIAGNOSING CANCER

The present invention also provides kits for use in diagnosing cancer, wherein the kit comprises an agent for detecting an LRRN1 antibody or measuring the LRRN1 antibody titre. In one embodiment, the LRRN1 antibody is an antibody that binds to a polypeptide comprising an amino acid sequence at least 90% homologous to SEQ ID NO:1. In another embodiment, the LRRN1 antibody is an antibody that binds to a polypeptide comprising an amino acid sequence at least 90% homologous to SEQ ID NO:2.

In one embodiment, the kit further comprises a label indicates that the agent for measuring the antibody level is for detecting cancer, wherein the antibody level in the test sample is higher, relative to the antibody level of a corresponding cancer-free sample, indicate the presence of cancer. In one embodiment, the cancer expresses LRRN1. In another embodiment, the cancer is selected from pancreatic cancer, cholangiocarcinoma, gall bladder cancer or breast cancer.

In one embodiment, the kit further comprises an agent for identifying CA19-9 for diagnosing pancreatic cancer and pancreatitis. In another embodiment, the kit further comprises an agent for identifying CA15-3 for diagnosing breast cancer.

In another embodiment, the agent is an immunoassay. The agent can be an agent known in the art for measuring the expression level of a specific, or any antibody, or a specific biomarker.

The present invention also provides kits for use in diagnosing pancreatitis, wherein the kit comprises an agent for detecting an LRRN1 antibody or measuring the LRRN1 antibody titre..In one embodiment, the kit further comprises a label indicates that the agent for measuring the antibody level is for detecting cancer.

Embodiments of the present invention are illustrated by the following examples, which are not to be construed in any way as imposing limitations upon the scope thereofDuring the studies described in the following examples, conventional procedures were followed, unless otherwise stated. Some of the procedures are described below for illustrative purpose.

### Example 1: Preparing Recombinant LRRN1 From Human Embryonic Stem Cells

Full-length human LRRN1 was amplified from human embryonic stem cells (HES-5) cDNA, using PCR method and the following specific primers: a) a forward primer nucleotide sequence (GATCGGATCCATGGCTAGGATGAGCTTTGTTATAGCA, SEQ ID NO: 3) and b) a reverse primer nucleotide sequence (GATCCTCGAGTTACCACATGTAATAGCTTCTGGATGTGT, SEQ ID NO:4). The PCR product comprises 716 amino acid (identical to SEQ ID NO:2) and was constructed into pLKO AS3W puro vector (National RNAi Core Facility).

The extracellular domain of LRRN1, obtained from the full-length human LRRN1, was subcloned in pEF1/Myc-His plasmid (Invitrogen, USA) encoding 6 Histadine (6His) with the following primers: sense primer (GATCGGATCCATGGCTAGGATGAGCTTTGTTATAGCA, SEQ ID NO:5) and antisense primer (GATC-CTCGAGAAGGGCTGTACTGGTTTCTTGATCAG, SEQ ID NO:6). The amino acid sequence of the extracellular domain of human LRRN1 is identical to SEQ ID NO:1.

The construct of the extracellular domain of LRRN1 was transfected and expressed in 293F cells (FreeStyle™ 293-F Cells, Invitrogen, USA). Recombinant extracellular domain of LRRN1 in culture supernatant was purified with Histrap Excel column (GE Healthcare Biosciences, USA) and ÄKTA protein purification systems (Akta Avant25, GE Healthcare Biosciences, USA), and eluted using elution buffer (20 mM Tris-Hcl pH7.4, 100mM NaCl and 100mM imidazole). The purified recombinant extracellular domain of human LRRN1 was confirmed by sodium dodecyl sulfate-polyacrylamide gel electrophoresis (SDS-PAGE) and its concentration was determined at OD 280nm.

### Example 2: The Detection of LRRN1 Antibody in Subjects with Pancreatic Cancer Using Indirect ELISA Assay

Blood samples were collected from 40 subjects with pancreatic cancer, 14 subjects with chronic pancreatitis and 40 cancer free subjects (the control group). Aliquots of these samples were stored at -80°C until use. This research followed the tenets of the Declaration of Helsinki and written informed consent was obtained prior to blood collection.

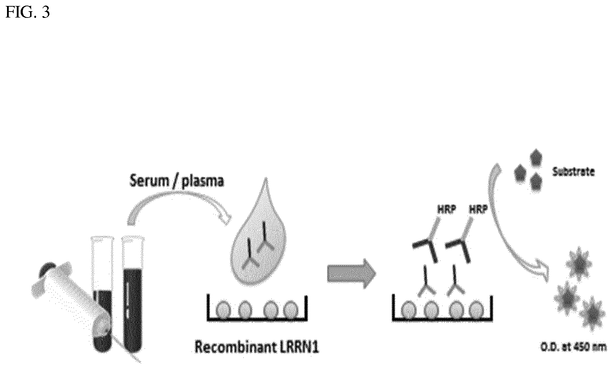

Using the glycosylated extracellular domain of LRRN1 protein (SEQ ID NO.:1) prepared in Example 1, the IgG antibody titer of the sera of cancer subjects, chronic pancreatitis subjects and the control group was measured by indirect ELISA, as illustrated in Fig. 3. CA 19-9 was also measured in the sera of cancer subjects, chronic pancreatitis subjects and control group (by a kit commercially available from Alpha Diagnostic, USA).

As for immobilization of the prepared protein on a solid phase, 100 *µ*g/mL per well of the recombinant extracellular domain of LRRN1 protein and carbonate buffer (pH8.6) was added to a Nunc C96 Maxisorp plate (Fisher, USA). The plate was left to stand at 4°C overnight and then washed twice in ddH₂O. As for blocking, PBS-0.05% Tween (Wako Pure Chemicals, Japan) with 3% BSA (bovine serum albumin, Sigma Aldrich, USA) was added to the plate, and the plate was shaken at 37°C for 1 hour. Serum samples of the pancreatic cancer subjects, chronic pancreatitis subjects and the control group were 1000-fold diluted with the blocking solution, and added to the plate. After washing the wells 5 times with phosphate buffered saline containing PBS-0.05% Tween, 1:5000 goat antihuman IgG, (Fab')₂-HRP secondary antibody was added (Jackson Immuno Research, USA) thereto, and the plate was shaken at 37°C for 1 hour to allow the reaction to proceed. After washing the wells 3 times with PBS-T, TMB (1- TMB (tetramethylbenzidine, eBioscience, USA) was added thereto, and the enzyme-substrate reaction was allowed to proceed. Thereafter, the reaction was stopped by adding IN H₂SO₄ (commercially available from Mallinckrodt Chemicals, USA), and then the absorbance was measured at 450 nm with a microplate reader.

### Results

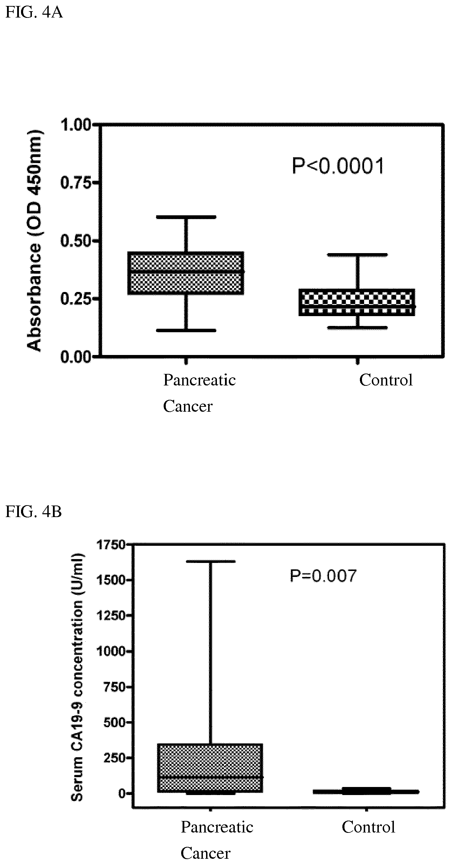

As shown in Tables 1 and 2 and Fig. 4A, sera from pancreatic cancer subjects show a significantly high antibody titer against the recombinant LRRN1 protein and a significantly high CA 19-9 level compare to the control subjects (P values for independent t-test, Wilcoxon rank-sum test, and Kruskall-wallist test were less than 0.05). Similarly, sera from chronic pancreatitis subjects show a significantly high antibody titer against the recombinant LRRN1 protein compare to normal subjects (P values for independent t-test, Wilcoxon rank-sum test, and Kruskall-wallist test were less than 0.05), see Tables 1 and 2, and Fig. 4B.

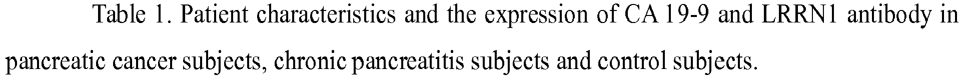

+

**Table 1. Patient characteristics and the expression of CA 19-9 and LRRN1 antibody in pancreatic cancer subjects, chronic pancreatitis subjects and control subjects.**

| | | **CA19-9** | | **LRRN1 AutoAbs** | |
|---|---|---|---|---|---|
| **Variable** | **N** | **Mean ± SD** | **Median** | **Mean ± SD** | **Median** |
| **Disease** | | | | | |
| **Normal** | 40 (42.55%) | 22.50 ± 44.81 | 12.50 | 0.2340 ± 0.0763 | 0.2159 |
| **Pancreas cancer** | 40 (42.55%) | 311.75 ± 589.08 | 123.50 | 0.3629 ± 0.1245 | 0.3670 |
| **Chronic pancreatitis** | 14 (14.90%) | 46.57 ± 59.07 | 20.00 | 0.5585 ± 0.2801 | 0.4948 |
| **Gender** | | | | | |
| **Male** | 58 (61.70%) | 185.88 ± 509.13 | 23.50 | 0.3395 ± 0.1877 | 0.3099 |
| **Female** | 36 (38.30%) | 90.03 ± 122.40 | 28.00 | 0.3335 ± 0.1673 | 0.2884 |
| **Survival** | | | | | |
| **Alive** | 41 (75.93%) | 188.46 ± 340.44 | 57.00 | 0.4141 ± 0.2158 | 0.3810 |
| **Dead** | 13 (24.07%) | 415.00 ± 873.97 | 146.00 | 0.4123 ± 0.1149 | 0.4060 |
| **Regional lymph node involvement** | | | | | |
| **No** | 21 (52.50%) | 179.48 ± 307.52 | 57.00 | 0.3699 ± 0.1319 | 0.3730 |
| **Yes** | 19 (47.50%) | 457.95 ± 777.05 | 214.00 | 0.3552 ± 0.1190 | 0.3195 |
| **Stage** | | | | | |
| **0** | 1 (2.50%) | 130.00 | 130.00 | 0.1425 | 0.1425 |
| **1** | 5 (12.50%) | 89.00 ± 74.56 | 99.00 | 0.4586 ± 0.0961 | 0.4703 |
| **2** | 31 (77.50%) | 364.94 ± 658.34 | 126.00 | 0.3581 ± 0.1217 | 0.3610 |
| **3** | 2 (5.00%) | 218.00 ± 308.30 | 218.00 | 0.3660 ± 0.1230 | 0.3360 |
| **4** | 1 (2.50%) | 146.00 | 146.00 | 0.2493 | 0.2493 |

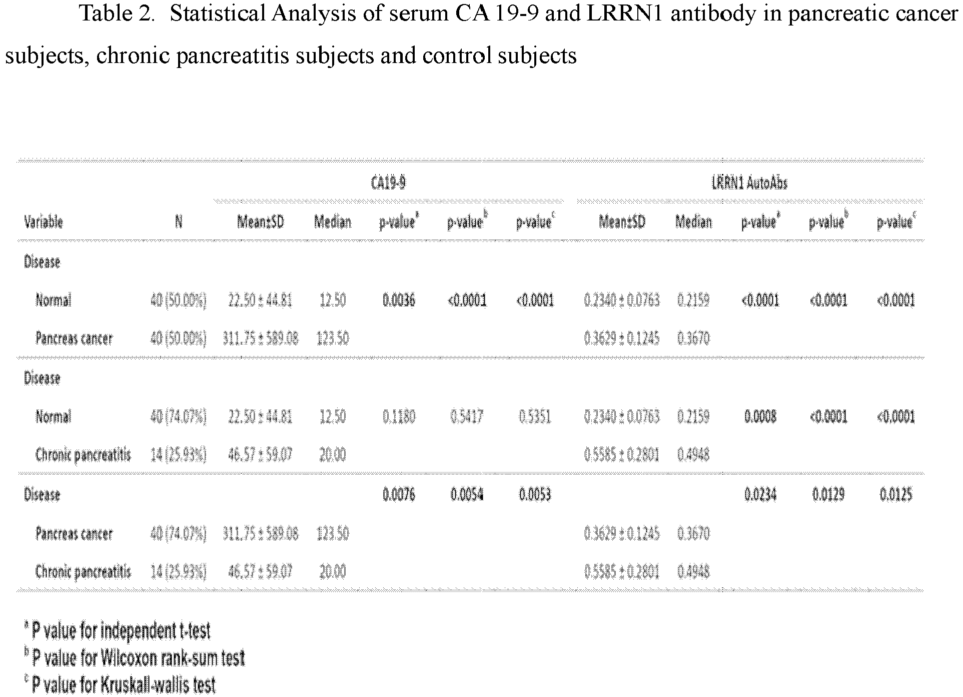

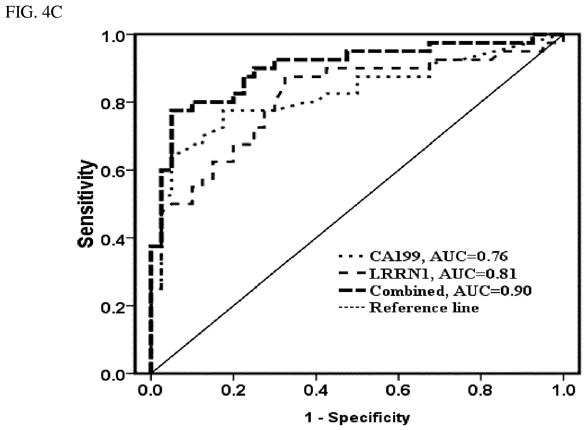

Fig. 4C shows the AUC for pancreatic cancer diagnosis is 0.81 for LRRN1 antibody 0.76 for CA 19-9 and 0.90 for the combination of LRRN1 antibody and CA 19-9. Advantageously, this combination has additive or synergistic effects on pancreatic cancer diagnosis. As illustrated in Table 3, a cutoff value for CA 19-9 at 39 can differentiate normal subjects and pancreatitis subjects, with a sensitivity of 0.43 and a specificity of 0.9 (P<0.0061 by Fisher's exact test or logistic regression) (Table 3). A cutoff value for LRRN1 antibody at 0.3493 can also diagnose pancreatitis, with a sensitivity of 0.79 and a specificity of 0.9 (p value <0.0001).

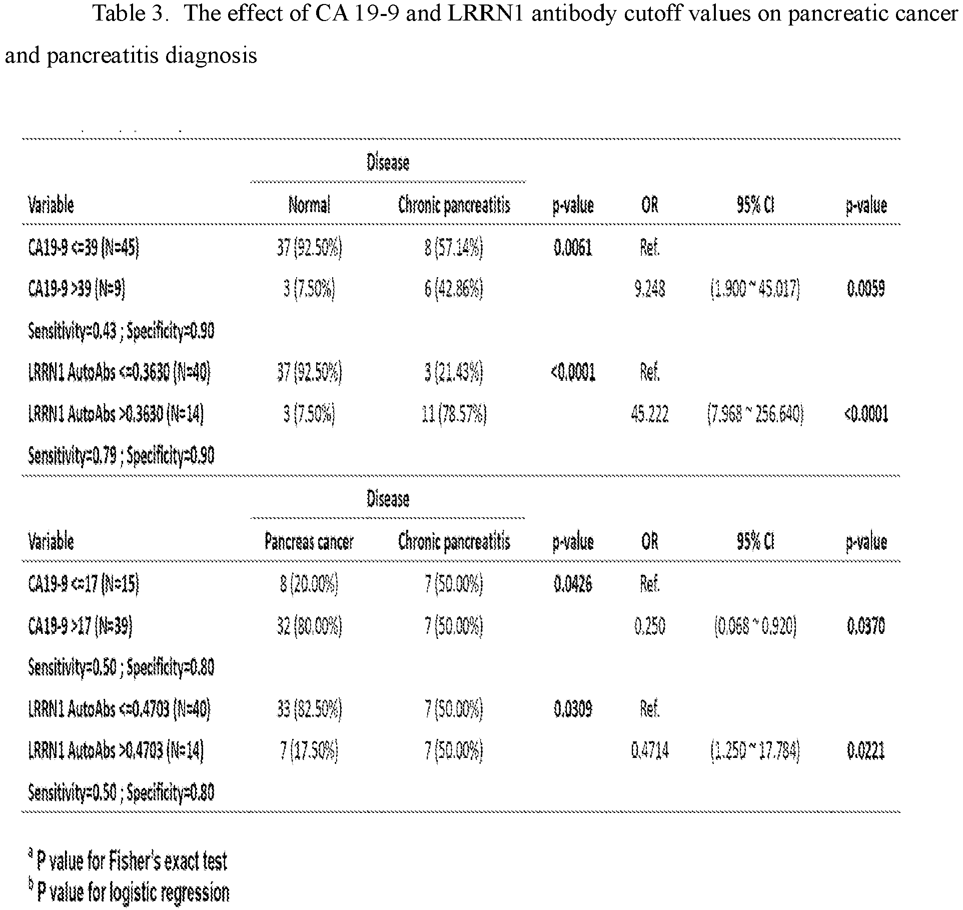

### Example 3: The Detection of LRRN1 Antibody in Subjects with Cholangiocarcinoma and Gall Bladder Cancer Using Indirect ELISA Assay

Blood samples were collected from 38 subjects with cholangiocarcinoma, 6 subjects with gall bladder cancer and 45 healthy subjects.

The LRRN1 antibody titre in these subjects was measured following the steps in Example 2.

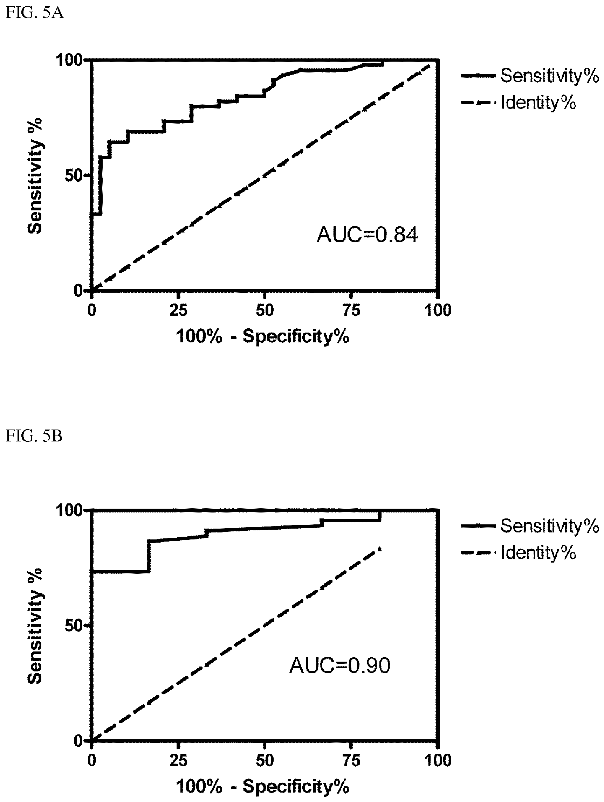

Results: Fig.5A shows the AUC for diagnosing cholangiocarcinoma is 0.84, with a sensitivity of 68% and a specificity of 89%. Fig. 5B shows the AUC for diagnosing gall bladder cancer is 0.90, with a sensitivity of 86% and a specificity of 83%.

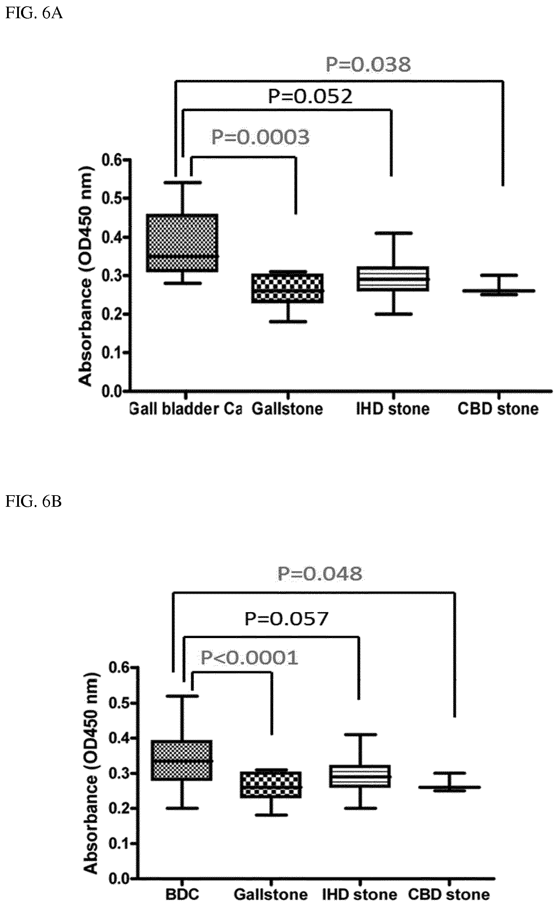

Fig 6A shows LRRN1 antibody tire could differentiate gall bladder carcinoma from gall bladder stone or common bile duct stone (AUC =0.93 and 0.94, separately). Fig. 6B shows LRRN1 antibody titre could differentiate subjects with bile duct stone or gall bladder stone from bile duct cancer (cholangiocarcinoma), with the AUC being 0.85 (bile duct stone vs. cholangiocarcinoma) and 0.84 (gall bladder stone vs. cholangiocarcinoma).

### Example 4: The Detection of LRRN1 Antibody in Subjects with Breast Cancer Using Indirect ELISA Assay

Blood samples were collected from 20 subjects with breast cancer and 20 healthy subjects. The LRRN1 antibody titre in these subjects was measured following the steps in Example 2.

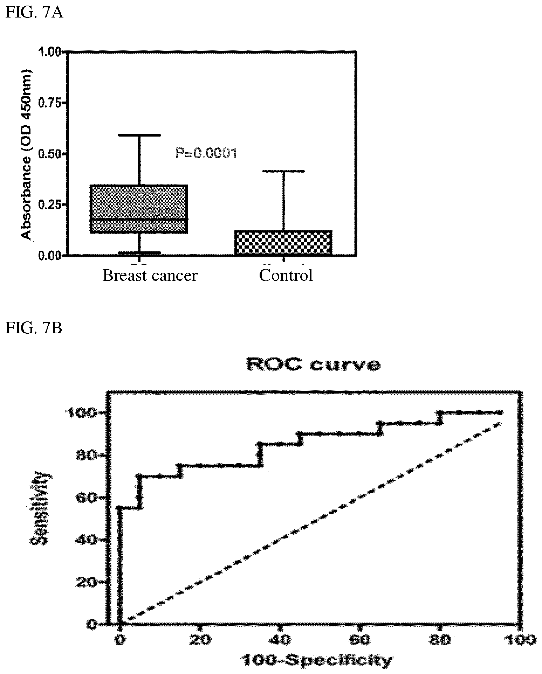

Results: Fig. 7A shows the LRRN1 antibody titre is significantly higher in patients with breast cancer compare to control and the AUC for breast cancer diagnosis using LRRN1 antibody is 0.8550, see Fig. 7B.

### Example 5: The Detection of LRRN1 Antibody and CA 15-3 in Subjects with Breast Cancer

Blood samples were collected from 124 subjects with different stages of breast cancer and 28 healthy subjects (control). The LRRN1 antibody titre in these subjects was measured following the steps in Example 2 and serum CA-15-3 level was measured using an ELISA kit (Human CA15-3 ELISA Kit, commercially available from RayBiotech, Inc., USA).

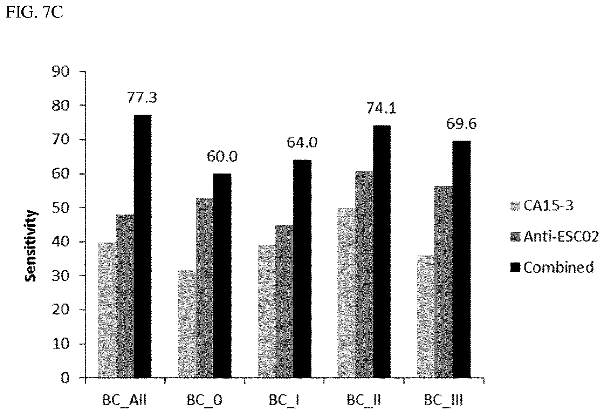

Results: Referring to Fig. 7C, the sensitivity of CA15-3 and LRRN1 antibody titre for diagnosing breast cancer of all stages is 40% and 48%, respectively. The sensitivity of the combination of CA15-3 and LRRN1 antibody titre for breast cancer diagnosis is 77% for breast cancer patients of all stages.

## Claims

1. An in vitro method for detecting cancer in a subject, comprising the steps of:
measuring the level of an antibody that binds to a polypeptide in the sample of the subject, wherein the polypeptide comprises an amino acid sequence at least 90% homologous to SEQ ID NO:1, and a higher antibody level in the subject, relative to the antibody level in a cancer free sample, is indicative the subject having cancer.

2. The method of claim 1, wherein the cancer expresses LRRN1.

3. The method of claim 1, wherein the cancer is pancreatic cancer.

4. The method of claim 3, further comprising the step of measuring CA19-9.

5. The method of claim 1, wherein the cancer is cholangiocarcinoma.

6. The method of claim 1, wherein the cancer is gallbladder cancer.

7. The method of claim 1, wherein the cancer is breast cancer.

8. The method of claim 7, further comprising the step of measuring CA 15-3.

9. An in vitro method for detecting pancreatitis in a subject, comprising the steps of:
measuring the level of an antibody that binds to a polypeptide in the sample of the subject, wherein the polypeptide comprises an amino acid sequence at least 90% homologous to SEQ ID NO:1, and a higher antibody level in the subject, relative to the antibody level in a pancreatitis free sample, is indicative the subject having pancreatitis.

10. The method of claim 9, wherein the polypeptide is leucine-rich repeat neuronal protein 1 (LRRN1).

11. The method of claim 9 or 10, further comprising the step of measuring amylase, lipase or the combination thereof.

12. The method of any of claims 1 to 11, wherein the antibody is measured by an immunoassay, preferably an enzyme linked immunosorbent assay or a radioimmunoassy.

13. A kit for detecting cancer, comprising
(a) An agent for measuring an antibody that binds to a polypeptide, wherein the polypeptide comprises an amino acid sequence at least 90% homologous to SEQ ID NO:1;
(b) a label indicates that the agent for measuring the antibody is for detecting cancer in a subject.

14. The kit of claim 13, wherein the antibody is selected from a polyclonal antibody, a monoclonal antibody, an immunoglobulin, or an antigen binding fragment thereof.

15. The kit of claim 13 or 14, wherein the antibody is IgG.
